# EUROPEAN PATENT APPLICATION

(11) **EP 4 635 499 A1**
(43) Date of publication of application: **22.10.2025**
(21) Application number: 23903590.0
(22) Date of filing: 15.12.2023
(51) Int. Cl.: A61K 31/5377, A61P 9/00, A61P 35/00, A61P 43/00

(54) **PHARMACEUTICAL COMPOSITION FOR VASCULAR MALFORMATIONS AND METHOD FOR CURING VASCULAR MALFORMATIONS**

(30) Priority: 16.12.2022 JP 2022201377
(71) Applicant: ARTham Therapeutics Inc., Tokyo 113-8650 (JP)
(72) Inventor: NAGABUKURO, Hiroshi, Yokohama-shi, Kanagawa 231-0023 (JP); TANAKA, Akira, Yokohama-shi, Kanagawa 231-0023 (JP); KUNIEDA, Kanako, Yokohama-shi, Kanagawa 231-0023 (JP)
(74) Representative: Vossius & Partner Patentanwälte Rechtsanwälte mbB
(86) International application number: PCT/JP2023/045011
(87) International publication number: WO 2024/128311

(57) **Abstract**

A pharmaceutical composition for managing or treating vascular malformations is provided that contains serabelisib or a pharmaceutically acceptable salt thereof as an active ingredientd, wherein a daily dose of the serabelisib is in a range from 50 mg to 100 mg.

## Description

### Technical Field

The present invention relates to a serabelisib-containing pharmaceutical composition for vascular malformations and to a method for treating vascular malformations using serabelisib.

### Background Art

Vascular malformations are diseases in which abnormalities are observed in the formation of blood vessels or lymphatic vessels. Vascular malformations do not undergo spontaneous regression and progress accompanying, e.g., growth, and are thus known as diseases that require lifelong disease treatment and management. Vascular malformations are classified according to, inter alia, the sites of lesions and are referred to as slow-flow vascular malformations when the malformations are observed in veins or lymphatic vessels. Slow-flow vascular malformations are classified into, e.g., venous malformations (VM) and lymphatic malformations (LM). Malformations observed over multiple combination of veins, arteries, capillaries, and lymphatic vessels are called mixed vascular malformations. Mixed vascular malformations are classified into, e.g., Klippel-Trenaunay syndrome (KTS) and Parkes-Weber syndrome. Within the PI3K pathway, which plays an important role in cell functions such as cell growth, proliferation, survival, motility, and metabolism, gain-of-function mutations have been reported in genes encoding PI3Kα and upstream and downstream kinases thereof in patients with the diseases above, suggesting PI3K pathway activation as a mechanism of disease onset.

The 2017 Guidelines for the Treatment of Hemangiomas, Vascular Malformations, and Lymphatic Malformations describe conservative and invasive treatments for the treatment of venous malformations, but do not describe any drug therapies. Surgical treatments such as resection, sclerotherapy with sclerosants, and medical management are carried out for the treatment of lymphatic malformations, and therapeutics such as sirolimus (mTOR inhibitor) have been reported to exhibit efficacies (NPL 1).

At the present time, sirolimus has received regulatory approval in Japan for the treatment of lymphangioleiomyomatosis and refractory lymphatic diseases, and alpelisib has received expedited approval in the United States for the treatment of PROS (PIK3CA Related Overgrowth Spectrum) (NPL 2 and NPL 3).

The PI3K pathway, which has been suggested to be deeply involved in the development of vascular malformations, is known to include PI3K, AKT, and mTOR as *in vivo* proteins, and inhibitors of these proteins have also been researched and developed as anticancer drugs. Existing inhibitors of these molecular targets have been reported to frequently cause hyperglycemia, which is considered to be a side effect in common with PI3K pathway inhibitors since hyperglycemia is caused by inhibitory actions on the PI3K pathway (NPL 4).

Since vascular malformations are diseases that may require long-term drug treatment, adverse events (side effects) associated with the pharmacological activity of a drug used in drug treatment may significantly impair the quality of life (QOL) of patients.

In practice, the following side effects are known for the existing therapeutic agents for vascular malformations.
Sirolimus (mTOR inhibitor): immunosuppression, hyperglycemia (unknown frequency) (NPL 2)
Alpelisib (PI3Kα inhibitor): hyperglycemia (NPL 3)

As described above, the existing treatment methods and therapeutic agents for vascular malformations have the problem of causing side effects such as hyperglycemia at therapeutic doses, and there is a need for drugs of which the doses causing side effects are different from those exhibiting therapeutic efficacies. However, as mentioned above, since it has been suggested that side effects such as hyperglycemia are related to the PI3Kα inhibitory activity, it is thought that the frequency of side effects will also increase in proportion to the efficacy of the drug. It is therefore entirely unclear as to whether there exists a drug, or a dosage therefor, at which the efficacy of the drug can be dissociated from side effects such as hyperglycemia.

Serabelisib is a compound with PI3Kα inhibitory activity and, by inhibiting the signal transduction pathway of the activated PI3K pathway, is expected to inhibit abnormal vascular formation and hyperplasia of bone and soft tissue and thus exhibit a therapeutic effect on vascular malformations.

Serabelisib is a compound represented by formula (I) below: 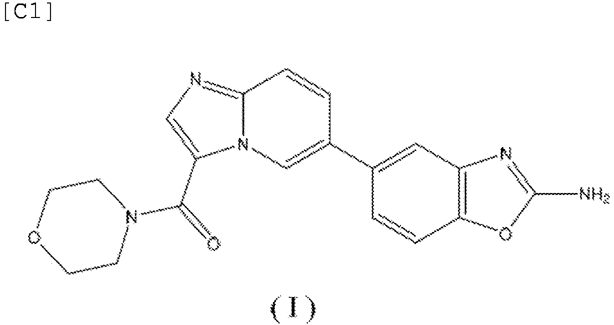 (While "serabelisib" and "ART-001" are referenced in the following, both of these represent the same compound.).

PTL 1 discloses that serabelisib has an excellent PI3Kα inhibitory activity and can be used to treat PI3Kα-mediated pathologies. PTL 2 discloses pharmaceutical compositions containing serabelisib as an active ingredient. NPL 5 discloses results related to the safety and tolerability of a single administration of serabelisib in humans.

However, there have been no disclosures or suggestions as to whether such a pharmaceutical composition containing serabelisib as an active ingredient actually exhibits therapeutic efficacy in actual vascular malformation patients and whether such a therapeutic efficacy, if any, is compatible with safety such as side effects.

### Citation List

### Patent Literature

PTL 1: WO 2011/022439 A
PTL 2: WO 2022/004859 A

### Non Patent Literature

NPL 1: "2017 Guidelines for the Treatment of Hemangiomas, Vascular Malformations, and Lymphatic Malformations", 2017, Section for "Research and Investigation of Refractory Hemangiomas, Vascular Malformations, Lymphangiomas, Complex Lymphatic Anomalies, and Related Diseases", Ministry of Health, Labour, and Welfare Research Grant, Policy Research Project for Intractable Diseases, Fiscal 2014-2016
NPL 2: Rapalimus (registered trademark) package insert, 2021 (revised), Nobelpharma Co., Ltd.
NPL 3: HIGHLIGHTS OF PRESCRIBING INFORMATION, "VIJOICE (registered trademark) (alpelisib) tablets, for oral use", 2019, U.S. Food and Drug Administration
NPL 4: "Complications of hyperglycaemia with PI3K-AKT-mTOR inhibitors in patients with advanced solid tumours on Phase I clinical trials, British Journal of Cancer", 2015, 113:1541-1547
NPL 5: "SAFETY AND PHARMACOKINETICS OF NOVEL PI3Kalpha INHIBITOR ART-001 IN ORAL PEDIATRIC FORMULATION FOR THE TREATMENT OF VASCULAR MALFORMATIONS", American Society for Clinical Pharmacology and Therapeutics Annual Meeting, 2021, 122nd:Abs PII-041

### Summary of the Invention

### Technical Problem

To date, existing drugs for treating vascular malformations have not accomplished suppression of the occurrence of side effects such as hyperglycemia. In particular, even with alpelisib (US product name VIJOICE^{(registered trademark)}), which has the same mechanism of action as serabelisib (another PI3Kα inhibitor), the onset of hyperglycemia as a side effect has been reported as a precautionary note when using the drug.

One of the problems to be solved by the present invention is to achieve, in the treatment of vascular malformations using serabelisib as an active ingredient, both suppression of side effects and clinical efficacy for vascular malformations.

### Means of Solving the Problems

As a result of intensive study, the present inventors have found that, in the treatment of vascular malformations using serabelisib as an active ingredient, clinical efficacy for vascular malformations and suppression of side effects can both be achieved by limiting the daily dose of serabelisib to a range from 50 mg to 100 mg.

As mentioned above, since the side effect of hyperglycemia caused by alpelisib, a conventional vascular malformation therapeutic, is suggested to be connected to its PI3Kα inhibitory activity, the frequency of side effects is also thought to increase in proportion to the drug efficacy. Serabelisib, another PI3Kα inhibitor like alpelisib, is also thought to exert drug efficacy thereof based on the same mechanism of action. Nevertheless, the present inventors have found that there is a dose range for serabelisib that is effective in treating vascular malformations with, at the same time, suppressing hyperglycemia as a side effect. This is a very unexpected and surprising finding, given that existing therapeutics have the problem of causing hyperglycemia as a side effect. Furthermore, the present inventors have found that efficacy and safety are ensured even when long-term administration (24 weeks) is carried out at this dose, thereby completing the present invention.

Accordingly, the present invention encompasses the following inventions.
[Item 1] A pharmaceutical composition for managing or treating a vascular malformation, containing serabelisib or a pharmaceutically acceptable salt thereof as an active ingredient, wherein a daily dose of the serabelisib is in a range from 50 mg to 100 mg.
[Item 2] A pharmaceutical composition for shrinking a swelling lesion in a vascular malformation patient, containing serabelisib or a pharmaceutically acceptable salt thereof as an active ingredient, wherein a daily dose of the serabelisib is in a range from 50 mg to 100 mg.
[Item 3] A pharmaceutical composition for reducing pain in a vascular malformation patient, containing serabelisib or a pharmaceutically acceptable salt thereof as an active ingredient, wherein a daily dose of the serabelisib is in a range from 50 mg to 100 mg.
[Item 4] The pharmaceutical composition according to any one of items 1 to 3, which causes a volume of a target lesion during a treatment period or after treatment to be reduced by at least 20% in comparison to the volume of the target lesion before the treatment.
[Item 5] The pharmaceutical composition according to any one of items 1 to 4, which can treat a vascular malformation while suppressing the occurrence of a side effect that may occur during the treatment period.
[Item 6] The pharmaceutical composition according to item 5, wherein the side effect that may occur during the treatment period is hyperglycemia.
[Item 7] A pharmaceutical composition for managing or treating a vascular malformation, containing serabelisib or a pharmaceutically acceptable salt thereof as an active ingredient, wherein the pharmaceutical composition is administered at a dose that
   (a) causes a volume of a target lesion during a treatment period or after treatment to be reduced by at least 20% in comparison to the volume of the target lesion before the treatment, and also
   (b) does not cause a drug-induced side effect of hyperglycemia during the treatment period.
[Item 8] The pharmaceutical composition according to item 7, wherein a daily dose of the serabelisib is in a range from 50 mg to 100 mg.
[Item 9] The pharmaceutical composition according to any one of item 1 to item 8, wherein a serabelisib content is in a range from 2 w/w% to 40 w/w% with reference to a total amount of the composition.
[Item 10] The pharmaceutical composition according to item 9, wherein the serabelisib content is 20 w/w% with reference to the total amount of the composition.
[Item 11] The pharmaceutical composition according to any one of items 1 to 10, wherein the vascular malformation is slow-flow vascular malformation or mixed vascular malformation.
[Item 12] The pharmaceutical composition according to any one of items 1 to 11, wherein the vascular malformation is a venous malformation, a lymphatic malformation, or Klippel-Trenaunay syndrome.
[Item 13] The pharmaceutical composition according to item 12, wherein the vascular malformation is a venous malformation.
[Item 14] The pharmaceutical composition according to item 12, wherein the vascular malformation is a lymphatic malformation.
[Item 15] The pharmaceutical composition according to item 12, wherein the vascular malformation is Klippel-Trenaunay syndrome.
[Item 16] The pharmaceutical composition according to any one of items 1 to 15, which is administered once per day over a treatment period of at least 2 weeks.
[Item 17] The pharmaceutical composition according to item 16, which is administered once per day over a treatment period of at least 4 weeks, at least 12 weeks, at least 24 weeks, at least 36 weeks, at least 48 weeks, at least 52 weeks, at least 2 years, or at least 3 years.
[Item 18] A method for managing or treating a vascular malformation, including: administering, to a subject in need thereof, serabelisib or a pharmaceutically acceptable salt thereof as an active ingredient at a daily dose of the serabelisib in a range from 50 mg to 100 mg.
[Item 19] A method for shrinking a swelling lesion in a vascular malformation patient, including: administering, to a subject in need thereof, serabelisib or a pharmaceutically acceptable salt thereof as an active ingredient at a daily dose of the serabelisib in a range from 50 mg to 100 mg.
[Item 20] A method for reducing pain in a vascular malformation patient, including: administering, to a subject in need thereof, serabelisib or a pharmaceutically acceptable salt thereof as an active ingredient at a daily dose of serabelisib in a range from 50 mg to 100 mg.
[Item 21] The method according to any one of items 18 to 20, wherein a volume of a target lesion during a treatment period or after treatment is reduced by at least 20% in comparison to the volume of the target lesion before the treatment.
[Item 22] The method according to any one of item 18 to item 21, which can treat a slow-flow vascular malformation while suppressing the occurrence of a side effect that may occur during the treatment period.
[Item 23] The method according to item 22, wherein the side effect that may occur during the treatment period is hyperglycemia.
[Item 24] A method for managing or treating a vascular malformation, the method including: administering, to a subject in need thereof, serabelisib or a pharmaceutically acceptable salt thereof as an active ingredient at a dose that
   (a) causes a volume of a target lesion during a treatment period or after treatment to be reduced by at least 20% in comparison to the volume of the target lesion before the treatment, and also
   (b) does not cause a drug-induced side effect of hyperglycemia during the treatment period.
[Item 25] The method according to item 24, wherein a daily dose of serabelisib is in a range from 50 mg to 100 mg.
[Item 26] The method according to any one of items 18 to 25, wherein the vascular malformation is a slow-flow vascular malformation or a mixed vascular malformation.
[Item 27] The method according to any one of items 18 to 26, wherein the vascular malformation is a venous malformation, a lymphatic malformation, or Klippel-Trenaunay syndrome.
[Item 28] The method according to item 27, wherein the vascular malformation is a venous malformation.
[Item 29] The method according to item 27, wherein the vascular malformation is a lymphatic malformation.
[Item 30] The method according to item 27, wherein the vascular malformation is Klippel-Trenaunay syndrome.
[Item 31] The method according to any one of items 18 to 30, wherein the serabelisib is administered once per day over a treatment period of at least 2 weeks.
[Item 32] The method according to item 31, wherein the serabelisib is administered once per day over a treatment period of at least 4 weeks, at least 12 weeks, at least 24 weeks, at least 36 weeks, at least 48 weeks, at least 52 weeks, at least 2 years, or at least 3 years.

### Advantageous Effects of Invention

The present invention can achieve, in the treatment of vascular malformations using serabelisib as an active ingredient, both clinical efficacy for vascular malformations and suppression of side effects.

### Description of Embodiments

The present invention is described in detail in the following in accordance with specific embodiments. However, the present invention is not constrained by or to the following embodiments and may be executed in any form within a range that does not deviate from the scope of the present invention. Embodiments that combine any two or more of the various embodiments described in the following are also included within the scope of the present invention unless clearly incompatible in terms of definitions or context.

In the present Description, when a numerical range is indicated as, for example, "from A to B" or "A to B," it means "greater than or equal to A and less than or equal to B" with both the end values inclusive, unless particularly indicated otherwise.

In the present Description, the term "serabelisib" occurring by itself means "serabelisib or a pharmaceutically acceptable salt thereof", unless clearly contradicted by the context or unless particularly indicated otherwise. However, in the context where a pharmaceutically acceptable salt of serabelisib is used as an active ingredient, the serabelisib referenced in the phrase "daily dose of serabelisib" means serabelisib in its free form.

In the present Description, "treating" a "vascular malformation" denotes causing the volume of a target lesion during the treatment period or after the treatment to be reduced in comparison to that prior to the treatment. This preferably denotes bringing about at least a 20% reduction in the volume of the target lesion.

A first aspect of the present invention relates to a pharmaceutical composition having various indications with regard to vascular malformations, wherein the pharmaceutical composition contains serabelisib or a pharmaceutically acceptable salt thereof as an active ingredient.

The pharmaceutical composition according to an embodiment is a pharmaceutical composition for managing or treating a vascular malformation, that characteristically contains serabelisib or a pharmaceutically acceptable salt thereof as an active ingredient wherein the daily dose of serabelisib is in the range from 50 mg to 100 mg.

The pharmaceutical composition according to another embodiment is a pharmaceutical composition for shrinking a swelling lesion in a vascular malformation patient, that characteristically contains serabelisib or a pharmaceutically acceptable salt thereof as an active ingredient wherein the daily dose of serabelisib is in the range from 50 mg to 100 mg.

The pharmaceutical composition according to another embodiment is a pharmaceutical composition for reducing pain in a vascular malformation patient, that characteristically contains serabelisib or a pharmaceutically acceptable salt thereof as an active ingredient wherein the daily dose of serabelisib is in the range from 50 mg to 100 mg.

According to an embodiment, each of the preceding pharmaceutical compositions preferably causes the volume of a target lesion during the treatment period or after the treatment to be reduced by at least 20% in comparison to that prior to the treatment.

According to an embodiment, each of the preceding pharmaceutical compositions preferably treats a vascular malformation while suppressing the occurrence of a side effect that may occur during the treatment period. According to an embodiment, the vascular malformation that is treated while suppressing the occurrence of a side effect is preferably a slow-flow vascular malformation. According to an embodiment, preferably the side effect that may occur during the treatment period is hyperglycemia.

The pharmaceutical composition according to still another embodiment is a pharmaceutical composition for managing or treating a vascular malformation, wherein the pharmaceutical composition characteristically contains serabelisib or a pharmaceutically acceptable salt thereof as an active ingredient and is administered at a dose that both
(a) causes the volume of a target lesion during the treatment period or after the treatment to be reduced by at least 20% in comparison to that prior to the treatment, and
(b) does not cause a drug-induced side effect of hyperglycemia during the treatment period.

According to an embodiment, the daily dose of serabelisib for this drug composition is preferably in the range from 50 mg to 100 mg.

The serabelisib content in each of the preceding pharmaceutical compositions is not particularly limited. However, according to an embodiment, the serabelisib content in each of the preceding pharmaceutical compositions is, for example, in the range from 2 w/w% to 40 w/w%, or from 5 w/w% to 35 w/w%, or from 10 w/w% to 30 w/w%, or from 15 w/w% to 25 w/w%, or from 15 w/w% to 25 w/w%, with reference to the total amount of the composition. According to an embodiment, the serabelisib content in each of the preceding pharmaceutical compositions is 15 w/w%, 20 w/w%, or 25 w/w% with reference to the total amount of the composition. According to an embodiment, the serabelisib content in each of the preceding pharmaceutical compositions is 20 w/w% with reference to the total amount of the composition.

The dosage form for each of the preceding compositions is not particularly limited. However, according to an embodiment, each of the preceding pharmaceutical compositions is a dosage form that can be orally administered. According to an embodiment, each of the preceding pharmaceutical compositions can be a tablet, capsule, granule, powder, syrup, dry syrup, enteric-coated preparation, or a sustained-release preparation. Dry syrup is preferred among the preceding.

In addition to the serabelisib or pharmaceutically acceptable salt thereof functioning as an active ingredient, each of the preceding pharmaceutical compositions may additionally contain other freely selected components. Examples of such other components may include, but are not limited to: excipients such as mannitol, erythritol, powdered reduced maltose syrup, crystalline cellulose, and corn starch; disintegrants such as crospovidone, croscarmellose sodium, sodium starch glycolate, partially pregelatinized starch, carmellose calcium, and low-substituted hydroxypropyl cellulose; sweeteners such as sucralose, aspartame, acesulfame potassium, saccharin sodium, monoammonium glycyrrhizinate, and thaumatin; and other additives such as fluidizers, colorants, and flavors. These components can be selected and used as appropriate depending on, inter alia, the dosage form of the pharmaceutical composition. Any one of these components may be used alone, or two or more may be used in any combination and ratio.

The components and formulations that can be used for each of the preceding pharmaceutical compositions are not particularly limited, but for details reference can be made as appropriate to the descriptions in, for example, University of the Sciences in Philadelphia, "Remington: The Science and Practice of Pharmacy, 20th Edition", Lippincott Williams & Wilkins, 2000, and the descriptions in PTL 2.

A second aspect of the present invention relates to various methods related to vascular malformations, wherein the methods include administering, to a subject, serabelisib or a pharmaceutically acceptable salt thereof as an active ingredient.

A method according to an embodiment is a method for managing or treating a vascular malformation, wherein the method characteristically includes administering, to a subject in need thereof, serabelisib or a pharmaceutically acceptable salt thereof as an active ingredient at a daily dose of serabelisib in the range from 50 mg to 100 mg.

A method according to another embodiment is a method for shrinking a swelling lesion in a vascular malformation patient, wherein the method characteristically includes administering, to a subject in need thereof, serabelisib or a pharmaceutically acceptable salt thereof as an active ingredient at a daily dose of serabelisib in the range from 50 mg to 100 mg.

A method according to another embodiment is a method for reducing pain in a vascular malformation patient, wherein the method characteristically includes administering, to a subject in need thereof, serabelisib or a pharmaceutically acceptable salt thereof as an active ingredient at a daily dose of serabelisib in the range from 50 mg to 100 mg.

According to an embodiment, in each of the preceding methods the volume of a target lesion during the treatment period or after treatment is reduced by at least 20% in comparison to that before the treatment.

According to an embodiment, in each of the preceding methods preferably a slow-flow vascular malformation is treated while suppressing the occurrence of a side effect that may occur during the treatment period. According to an embodiment, preferably a side effect that may occur during the treatment period is hyperglycemia.

A method according to yet another embodiment is a method for managing or treating a vascular malformation, wherein the method is characterized by administering, to a subject in need thereof, serabelisib or a pharmaceutically acceptable salt thereof as an active ingredient at a dose that both
(a) causes the volume of a target lesion during the treatment period or after treatment to be reduced by at least 20% in comparison to that before the treatment, and
(b) does not cause a drug-induced side effect of hyperglycemia during the treatment period.

According to an embodiment, the daily dose of serabelisib in this method is preferably in the range from 50 mg to 100 mg.

A third aspect of the present invention relates to both the pharmaceutical composition that is the first aspect and the various methods that constitute the second aspect.

According to an embodiment, the vascular malformation is a slow-flow vascular malformation or a mixed vascular malformation. According to an embodiment, the vascular malformation is one or two or more diseases selected from venous malformations, lymphatic malformations, and Klippel-Trenaunay syndrome.

According to an embodiment, the side effects are hyperglycemia and hypercreatinemia. According to an embodiment, the side effect is hyperglycemia. In the present Description, "hyperglycemia" is an adverse event (side effect) associated with the pharmacological action of a drug and is diagnosed by a physician based on test values such as the blood glucose level.

According to an embodiment, there are no particular limitations on the subjects for the administration of the respective pharmaceutical compositions described in the preceding and for the administration of serabelisib by the respective methods. According to an embodiment, the subject for the administration of serabelisib is a human. According to an embodiment, the subject for the administration of serabelisib is an adult. When the subject for administration is an adult, the daily dose of serabelisib is in the range from 50 mg to 100 mg. According to an embodiment, the subject for the administration of serabelisib is a child. When the subject for administration is a child, the daily dose of serabelisib can be a pediatric dose proportional to the 50 mg to 100 mg adult dose range. Such a pediatric dose can be calculated based on body surface area according to the Crawford formula from the adult dose range of from 50 mg to 100 mg.

According to an embodiment, the administration of serabelisib using the respective pharmaceutical compositions described in the preceding and the respective methods described in the preceding may be carried out at any of the following times: prior to a meal, during a meal, and after a meal. According to an embodiment, the administration of serabelisib is preferably carried out after a meal. According to an embodiment, the administration of serabelisib is preferably carried out at least after breakfast.

The frequency of administrations of serabelisib using the respective pharmaceutical compositions described in the preceding and the respective methods described in the preceding is not particularly limited. It may be once a day, or administration may be carried out by dividing the daily amount into a plurality of administrations. According to an embodiment, serabelisib is administered once a day using the respective pharmaceutical compositions described in the preceding and the respective methods described in the preceding. According to another embodiment, the administration of serabelisib using the respective pharmaceutical compositions described in the preceding and the respective methods described in the preceding is carried out by dividing the daily amount into a plurality of administrations. According to an embodiment, the administration of serabelisib using the respective pharmaceutical compositions described in the preceding and the respective methods described in the preceding is preferably carried out once daily after breakfast.

The interval of administrations of serabelisib using the respective pharmaceutical compositions described in the preceding and the respective methods described in the preceding is not particularly limited. However, according to an embodiment, the interval of administrations of serabelisib using the respective pharmaceutical compositions described in the preceding and the respective methods described in the preceding is, for example, 6 to 48 hours, 8 to 36 hours, 12 to 36 hours, 20 to 28 hours, or 22 to 26 hours.

There are no particular limitations on the treatment period with serabelisib using the respective pharmaceutical compositions described in the preceding and the respective methods described in the preceding. However, according to an embodiment, the treatment period with serabelisib using the respective pharmaceutical compositions described in the preceding and the respective methods described in the preceding is, for example, at least 2 weeks, at least 4 weeks, at least 12 weeks, at least 24 weeks, at least 36 weeks, at least 48 weeks, at least 52 weeks, at least 2 years, or at least 3 years. According to an embodiment, the treatment period with serabelisib using the respective pharmaceutical compositions described in the preceding and the respective methods described in the preceding is, for example, at most 4 weeks, at most 12 weeks, at most 24 weeks, at most 36 weeks, at most 48 weeks, at most 52 weeks, at most 2 years, or at most 3 years. According to an embodiment, the respective pharmaceutical compositions described in the preceding and the respective methods described in the preceding exhibit the characteristic feature of ensuring both efficacy and safety even during long-term administration.

A fourth aspect of the present invention relates to the use of serabelisib or a pharmaceutically acceptable salt thereof in the manufacture of the respective pharmaceutical compositions described in the preceding. The details of each pharmaceutical composition are described in the preceding.

A fifth aspect of the present invention relates to serabelisib or a pharmaceutically acceptable salt thereof for use in the applications of the respective methods described in the preceding. The details of each method are described in the preceding.

### Examples

The present invention is described in more detail in accordance with the examples. However, the following examples are nothing more than examples that only conveniently illustrate details, and the present invention should not be limited by or to the following examples in any way.

### [Study example] Confirmation study of the efficacy and safety of serabelisib (ART-001) using vascular malformation patients as subjects

The efficacy was confirmed in a randomized double-blind study in vascular malformation patients. A serabelisib-containing dry syrup (contained 200 mg of serabelisib in 1 g) was administered orally for 24 weeks once a day after breakfast at an adult serabelisib dose of 50 mg or an adult serabelisib dose of 100 mg. For the pediatric patients, the administered dose was made the dose calculated based on body surface area conversion according to the Crawford formula for each of the adult doses. The primary endpoint was the response rate based on the volume change (measured by MRI) of the target lesion at the end of 24 weeks of administration.

In this study, "baseline" refers to each measurement value related to symptom severity that provides a reference prior to administration. The baseline is measured at a designated uniform interval prior to administration.

The number of days of administration of the pharmaceutical composition of serabelisib is the number of days using the baseline as the first day, and expressions such as the "administration period" and "number of weeks of administration" also conform to this standard. That is, the day of baseline measurement is the day on which administration of the pharmaceutical composition of serabelisib is begun.

### < Analysis related to efficacy >

### (1) Main analysis for efficacy

In the analysis of the primary endpoint for evaluating efficacy, a complete response was defined as the disappearance of the target lesion and a partial response was defined as an at least 20% reduction in the volume of the target lesion in comparison to that at the time of the screening test. The 95% confidence interval for the response rate (the sum of complete responses and partial responses as a percentage of the total) based on the change in target lesion volume (measured by MRI) between prior to administration and the time point of completion of 24 weeks of administration, was calculated using the Clopper-Pearson method, and whether its lower limit exceeded 12.5% was checked.

For the secondary endpoint, the compliance based on the change in target lesion volume (measured by MRI) at the time point of completion of 12 weeks of administration was evaluated.

### (2) Secondary analysis of efficacy

### · Pain evaluation:

The severity of pain at the baseline, week 4 of administration, week 12 of administration, and week 24 of administration was evaluated (using VAS).

### < Items investigated for efficacy >

The following items were investigated and their results were recorded.

### (1) Measurement of target lesion volume using MRI

### · Measurement method:

With respect to blood vessel malformation lesions, cystic components of lymphatic malformations, lymphedema, lymph fluid, and lymphorrhea (including also intraosseous, extraosseous, and subcutaneous), which are components of vascular malformations, the target lesion detected using the T2 fat-suppressed image was selected for the target lesion and its volume was measured.

### (2) Measurement of pain severity using the Visual Analogue Scale (VAS)

### · Measurement method:

A 10-cm straight line was drawn on the recording paper; the left end was defined as 0 and the right end was defined as 100; "0" was assigned to a condition of "pain completely absent" and "100" was assigned to a condition of "the worst pain, or a more severe pain cannot be imagined"; and inquiry was made as to where the current pain level was on the straight line and this was recorded. It was also recorded when the information about a subject was provided by someone else who was not the subject. In addition, the subjects were classified into under 15 years and 15 years and over for the age when consent was obtained, and recording paper appropriate for each age group was used.

### < Patient subjects and principal inclusion criteria >

The vascular malformation patients satisfied the following diagnostic criteria and conditions.
(1) Males and females at least 2 years of age.
(2) Patients diagnosed with venous malformations, lymphatic malformations, or Klippel-Trenaunay syndrome.
(3) Symptomatic patients.
(4) Patients assessed as refractory.

### < Principal exclusion criteria >

(1) Patients that have diabetes or a disease that causes glucose metabolic abnormalities.
(2) Patients with renal dysfunction.
(3) Patients with hepatic dysfunction.
(4) Patients with inadequately controlled ischemic heart disease, arrhythmia, or heart failure.
(5) Patients receiving a medicament that has a CYP3A4 inhibiting or inducing activity.

### < Main criteria for discontinuation >

(1) The study is discontinued when a test value of 140 mg/dL or higher is observed for the blood glucose level (fasting) in the clinical test performed at each visit.
(2) The study is discontinued when the clinical tests performed at each visit meets all of the following criteria stipulated for each age group.

### << 18 years and over >>

· An increase in serum creatinine of 1.5-times or more from the test value obtained on the day that administration of the study drug was begun.
· An increase in serum creatinine of 0.3 mg/dL or more from the test value obtained on the day that administration of the study drug was begun.
· An increase in serum cystatin C of 1.5-times or more from the test value obtained on the day that administration of the study drug was begun.
· The serum cystatin C exceeds 0.95 mg/L.

### << Under 18 years of age >>

· An increase in serum creatinine of 1.5-times or more from the test value obtained on the day that administration of the study drug was begun.
· An increase in serum cystatin C of 1.5-times or more from the test value obtained on the day that administration of the study drug was begun.
· The serum cystatin C exceeds 0.95 mg/L.

### < Study subject patients >

The investigational drug was assigned as follows to thirty-five vascular malformation patients, and data analysis was performed on these patient groups.

The group receiving dry syrup at a dose providing 50 mg of serabelisib was designated the "50 mg administration group", and the group receiving dry syrup at a dose providing 100 mg of serabelisib was designated the "100 mg administration group". 17 patients were assigned to the 50 mg administration group and 18 patients were assigned to the 100 mg administration group.

The two groups had comparable demographic characteristics.

### < Results for the primary and secondary endpoints for efficacy >

The table given below indicates, as primary endpoints: the response rates based on the change in the volume of the target lesion at the 24th week of administration and the numbers of subjects, and the 95% confidence intervals according to the Clopper-Pearson method. Also indicated in the following table as secondary endpoints are: the response rates at the 12th week of administration and the numbers of subjects.

**[Table 1]**

| ADMINISTRATION GROUP (NUMBER OF SUBJECTS) | 50mg ART-001(17) | 100mg ART-001(18) |
|---|---|---|
| RESPONSE RATE AT 12 WEEKS OF ADMINISTRATION (NUMBER OF SUBJECTS) | 0% (0) | 16.7% (3) |
| RESPONSE RATE AT 24 WEEKS OF ADMINISTRATION (NUMBER OF SUBJECTS) | 29.4% (6) | 33.3% (6) |
| 95% CONFIDENCE INTERVAL FOR RESPONSE RATE AT 24 WEEKS OF ADMINISTRATION | 10.3% - 56.0% | 13.3% - 59.0% |
| VOLUME CHANGE (AVERAGE VALUE) OF TARGET LESION AT 24 WEEKS OF ADMINISTRATION | -2.3% | -12.6% |

| ADMINISTRATION GROUP (NUMBER OF SUBJECTS) | 50mg ART-001(17) | 100mg ART-001(18) |
|---|---|---|
| RESPONSE RATE AT THE 12^{th} WEEK OF ADMINIATRATION (NUMBER OF SUBJECTS) | 0% (0) | 16.7% (3) |
| RESPONSE RATE AT THE 24^{th} WEEK OF ADMINIATRATION (NUMBER OF SUBJECTS) | 29.4% (5) | 33.3% (6) |
| 95% CONFIDENCE INTERVAL FOR RESPONSE RATE AT THE 24^{th} WEEK OF ADMINIATRATION | 10.3% - 56.0% | 13.3% - 59.0% |
| VOLUME CHANGE OF TARGET LESION AT THE 24^{th} WEEK OF ADMINISTRATION (AVERAGE VALUE) | -2.3% | -12.6% |

The percentage of subjects in whom efficacy was observed was 29.4% (5/17) in the 50 mg administration group and 33.3% (6/18) in the 100 mg administration group, with the Clopper-Pearson 95% confidence interval being 10.3% to 56.0% for the 50 mg administration group and 13.3% to 59.0% for the 100 mg administration group. The lower limit for the 95% confidence interval was greater than 12.5% in the 100 mg administration group.

### < Results for the pain-related secondary endpoint >

The VAS progressions over the baseline, at week 4 of administration, at week 12 of administration, and at week 24 of administration are indicated in the following table.

**[Table 2]**

| | | BASELINE | WEEK 4 OF ADMINISTRATION | WEEK 12 OF ADMINISTRATION | WEEK 24 OF ADMINISTRATION |
|---|---|---|---|---|---|
| 50 mg ADMINISTRATION GROUP | NUMBER OF SUBJECTS | 17 | 17 | 17 | 17 |
| | AVERAGE VALUE | 29.4 | 20.6 | 21.2 | 16.1 |
| | MEDIAN VALUE | 16.0 | 6.0 | 100 | 4.0 |
| | STANDARD DEVIATION | 31.4 | 26.3 | 24.2 | 25.8 |
| 100 mg ADMINISTRATION GROUP | NUMBER OF SUBJECTS | 18 | 17 | 17 | 17 |
| | AVERAGE VALUE | 24.6 | 15.8 | 15.7 | 12.4 |
| | MEDIAN VALUE | 20.0 | 2.0 | 2.0 | 8.0 |
| | STANDARD DEVIATION | 26.6 | 21.9 | 20.6 | 15.0 |

| | | BASELINE | WEEK 4 OF ADMINISTRATION | WEEK 12 OF ADMINISTRATION | WEEK 24 OF ADMINISTRATION |
|---|---|---|---|---|---|
| 50 mg ADMINISTRATION GROUP | NUMBER OF SUBJECTS | 17 | 17 | 17 | 17 |
| | AVERAGE VALUE | 29.4 | 20.6 | 21.2 | 16.1 |
| | MEDIAN VALUE | 16.0 | 6.0 | 10.0 | 4.0 |
| | STANDARD DEVIATION | 31.4 | 26.3 | 24.2 | 25.8 |
| 100 mg ADMINISTRATION GROUP | NUMBER OF SUBJECTS | 18 | 17 | 17 | 17 |
| | AVERAGE VALUE | 24.6 | 15.8 | 15.7 | 12.4 |
| | MEDIAN VALUE | 20.0 | 2.0 | 2.0 | 8.0 |
| | STANDARD DEVIATION | 26.6 | 21.9 | 20.6 | 15.0 |

The average values and median values of the VAS were reduced after the treatment for both the 50 mg administration group and the 100 mg administration group.

### < Hyperglycemia side effect >

In this study, no subject exhibited an event that was diagnosed as hyperglycemia by a physician based on test values such as a blood glucose value.

The preceding results have demonstrated that the serabelisib-containing pharmaceutical composition according to the present invention accomplishes therapeutic effects in the treatment of vascular malformations without causing the side effect of hyperglycemia, which has been a problem with existing drug therapies such as alpelisib.

### Industrial Applicability

According to the present invention, a serabelisib-containing pharmaceutical composition can be used with greater safety in the treatment of vascular malformations because it accomplishes a therapeutic effect without causing the side effect of hyperglycemia, which may occur at high frequencies in existing drug therapies.

## Claims

1. A pharmaceutical composition for managing or treating a vascular malformation, comprising serabelisib or a pharmaceutically acceptable salt thereof as an active ingredient,
wherein a daily dose of the serabelisib is in a range from 50 mg to 100 mg.

2. A pharmaceutical composition for shrinking a swelling lesion in a vascular malformation patient, comprising serabelisib or a pharmaceutically acceptable salt thereof as an active ingredient,
wherein a daily dose of the serabelisib is in a range from 50 mg to 100 mg.

3. A pharmaceutical composition for reducing pain in a vascular malformation patient, comprising serabelisib or a pharmaceutically acceptable salt thereof as an active ingredient,
wherein a daily dose of the serabelisib is in a range from 50 mg to 100 mg.

4. The pharmaceutical composition according to any one of claims 1 to 3, which causes a volume of a target lesion during a treatment period or after treatment to be reduced by at least 20% in comparison to the volume of the target lesion before the treatment.

5. The pharmaceutical composition according to any one of claims 1 to 4, which can treat a vascular malformation while suppressing the occurrence of a side effect that may occur during the treatment period.

6. The pharmaceutical composition according to claim 5, wherein the side effect that may occur during the treatment period is hyperglycemia.

7. A pharmaceutical composition for managing or treating vascular malformation, comprising serabelisib or a pharmaceutically acceptable salt thereof as an active ingredient, wherein the pharmaceutical composition is administered at a dose that
(a) causes a volume of a target lesion during a treatment period or after treatment to be reduced by at least 20% in comparison to the volume of the target lesion before the treatment, and also
(b) does not cause a drug-induced side effect of hyperglycemia during the treatment period.

8. The pharmaceutical composition according to claim 7, wherein a daily dose of the serabelisib is in a range from 50 mg to 100 mg.

9. The pharmaceutical composition according to any one of claim 1 to claim 8, wherein a serabelisib content is in a range from 2 w/w% to 40 w/w% with reference to a total amount of the composition.

10. The pharmaceutical composition according to claim 9, wherein the serabelisib content is 20 w/w% with reference to the total amount of the composition.

11. The pharmaceutical composition according to any one of claims 1 to 10, wherein the vascular malformation is a slow-flow vascular malformation or a mixed vascular malformation.

12. The pharmaceutical composition according to any one of claims 1 to 11, wherein the vascular malformation is a venous malformation, a lymphatic malformation, or Klippel-Trenaunay syndrome.

13. The pharmaceutical composition according to claim 12, wherein the vascular malformation is a venous malformation.

14. The pharmaceutical composition according to claim 12, wherein the vascular malformation is a lymphatic malformation.

15. The pharmaceutical composition according to claim 12, wherein the vascular malformation is Klippel-Trenaunay syndrome.

16. The pharmaceutical composition according to any one of claims 1 to 15, which is administered once per day over a treatment period of at least 2 weeks.

17. The pharmaceutical composition according to claim 16, which is administered once per day over a treatment period of at least 4 weeks, at least 12 weeks, at least 24 weeks, at least 36 weeks, at least 48 weeks, at least 52 weeks, at least 2 years, or at least 3 years.

18. A method for managing or treating a vascular malformation, comprising administering, to a subject in need thereof, serabelisib or a pharmaceutically acceptable salt thereof as an active ingredient at a daily dose of the serabelisib in a range from 50 mg to 100 mg.

19. A method for shrinking a swelling lesion in a vascular malformation patient, comprising administering, to a subject in need thereof, serabelisib or a pharmaceutically acceptable salt thereof as an active ingredient at a daily dose of the serabelisib in a range from 50 mg to 100 mg.

20. A method for reducing pain in a vascular malformation patient, comprising administering, to a subject in need thereof, serabelisib or a pharmaceutically acceptable salt thereof as an active ingredient at a daily dose of the serabelisib in a range from 50 mg to 100 mg.

21. A method for managing or treating a vascular malformation, comprising administering, to a subject in need thereof, serabelisib or a pharmaceutically acceptable salt thereof as an active ingredient at a dose that
(a) causes a volume of a target lesion during a treatment period or after treatment to be reduced by at least 20% in comparison to the volume of the target lesion before the treatment, and also
(b) does not cause a drug-induced side effect of hyperglycemia during the treatment period.
